(19) Europäisches Patentamt
European Patent Office
Office européen des brevets

(11) EP 2 902 411 A1

(12) **EUROPEAN PATENT APPLICATION**

(43) Date of publication:
**05.08.2015 Bulletin 2015/32**

(51) Int Cl.:
***C07K 14/415*** *(2006.01)* ***C12N 15/82*** *(2006.01)*

(21) Application number: **14305150.6**

(22) Date of filing: **04.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Biogemma**
**75001 Paris (FR)**

(72) Inventor: **The designation of the inventor has not yet been filed**

(74) Representative: **Flesselles, Bruno F.G.**
**BF IP**
**4, rue Ribera**
**75016 Paris (FR)**

(54) **Method for plant improvement**

(57) The invention relates to the field of plant improvement, in particular of the improvement of yield for plants, by using a transgene containing a BETL promoter driving expression of the IncW2 protein.

EP 2 902 411 A1

SEQUENCE LISTING

<110> BIOGEMMA

<120> METHOD FOR PLANT IMPROVEMENT

<130> BRV 76 - EP PRIO

<160> 6

<170> PatentIn version 3.3

<210> 1
<211> 1911
<212> DNA
<213> Zea mays

<400> 1
ctcgagttac tcatgatggt catctaggat catagaccat ccccacagac caacatgagt 60

cttttctacg cactttgttc actcgtgtgc atcaaagaaa acttcttggt tggtcactca 120

tccaaaaatt gctctgagcc aagcatgctt atcttagagg ttttttgag ataagcttct 180

gaaaaagaag gtgcaccttg tttgtatgag tattatacca ttcctattaa gccttggaca 240

aagatatcac aatccaccta ggccaagata tcacatttcc caacttagtc tataaaagga 300

ctagacaaga catctcctta gaagagaagc cctacctctt gtgcccataa caggcacctc 360

caacttgaga actaatttca caaagagtca cgctcttggg aactccatgt actcatatgt 420

acacactaca tctaatgcat agaaacacca agatcacatt gtactagcaa aatgtcatag 480

aagactagtt aaaaccttgt ttggtctgct caaacttaac aaatcaccta ggagacatgc 540

tagaagtatc tcaacagaga atgacaccat atagtagtgg caccaagtgc ctaatctgca 600

cacaaaaaaa tcgtaccata catgacatca aggcttaata atagagtgta tgttaaagcg 660

agcatgcaac ctatgagtgg tatgtaggag ttaggtttaa acaaggtaat ggctcaagca 720

ccacacatcc taccacaatg tcgtaataaa tataaaagca ctagcaatct atttagcatg 780

cctaaatggg atactatgag gttgggtggg atgtggcacc tttgtataat ggcccagttc 840

cttagtgtag tcttgatcct ccccgttatg ttgagactcc tctagggatt ttgtaggaat 900

catcaaattt tcataagcaa tttcttgtgc acaaagaacc aaatagattg aaaaagtttc 960

aaattcactc aaacacaaaa ccatggcaca tagcttatgt gacaaaatat ttgggacact 1020

agtttcatat tttttgagat catataagtt tattatcaaa ctcccaagga ttaaattatt 1080

ttttgaaaaa aaagaaaaaa gggaaaacat cataaggtga cacatggcaa cctctgaatg 1140

actagacttt taccatctct caggtgggtc tggtcaacaa tcactgttgg tcggtcctta 1200

ccttgcctag acgggtcctt agtaggccta ctgggttgag ttatgggata aattgtggcc 1260

tagaaacata ccagtccacc aaccttggga ccacttaaaa aattgcatct ttcaccatta 1320

tactatttag atgtttttaa aaaacaatca taacttttac atcgaaatca aaactagaca 1380

aattttatac tttcacagag cagcagaaat ttatacaata tgattgaata caagatgtag 1440

gacccaatgg agagaatttt ttgtctccta tatgcttgaa tacccaacat aatatcttcg 1500

caacatacta tctatctaat agaaaaatta taatatagtt aaatacttaa gtagtatcta 1560

gtggatagaa ttcaatatct cctacatgca tgaggagtaa tatctactag acatgcaaca 1620

tatttttatc tatctaatag aatatatata ataaagttaa atattatatg catcacctac 1680

tatatataat ttgatatctt ttagatgtat aagggactaa gaataatatc tctagcacac 1740

atgcaatgca ttatctatct aaatatatta tataatagtt aaatattaat tatacgtagt 1800

ctaaacctac atataagcct acccatcccc acttaaagat ctcagtgtca cacatagacc 1860

atacatctca cttcgccaaa aaaattccgt caacagttga agttataccc t 1911

<210> 2
<211> 594
<212> PRT
<213> Zea mays

<400> 2

Met Arg Ala Leu Val Val Val Ser Phe Ala Ser Ala Cys Leu Leu Leu
1 5 10 15

Leu Leu Gln Leu Ala Gly Ala Ser His Val Val Tyr Asn Tyr Lys Asp
20 25 30

Leu Glu Ala Glu Ala Ala Ala Ala Thr Asp Gln Val Pro Pro Ser Ile
35 40 45

Val Asn Pro Leu Leu Arg Thr Gly Tyr His Phe Gln Pro Pro Lys Asn
50 55 60

Trp Ile Asn Asp Pro Asn Ala Pro Met Tyr Tyr Lys Gly Trp Tyr His
65 70 75 80

Phe Phe Tyr Gln Tyr Asn Pro Lys Gly Ala Val Trp Gly Asn Ile Val
85 90 95

Trp Ala His Ser Val Ser Arg Asp Leu Ile Asn Trp Val Ala Leu Glu
100 105 110

Pro Ala Leu Arg Pro Ser Ile Pro Gly Asp Arg Tyr Gly Cys Trp Ser
115 120 125

Gly Ser Ala Thr Val Leu Pro Asp Gly Gly Gly Pro Val Ile Met Tyr
130 135 140

Thr Gly Val Asp His Pro Asp Ile Asn Tyr Gln Val Gln Asn Val Ala
145 150 155 160

```
Tyr Pro Lys Asn Val Ser Asp Pro Leu Leu Arg Glu Trp Val Lys Pro
                165                 170                 175

Ser His Asn Pro Val Ile Val Pro Glu Gly Gly Ile Asn Ala Thr Gln
            180                 185                 190

Phe Arg Asp Pro Thr Thr Ala Trp Arg Gly Pro Gly His Glu Gln Trp
        195                 200                 205

Arg Leu Leu Val Gly Ser Ala Ala Gly Ser Ser Pro Pro Arg Gly Val
    210                 215                 220

Ala Tyr Val Tyr Arg Ser Arg Asp Phe Arg Arg Trp Arg Arg Val Gln
225                 230                 235                 240

Arg Pro Leu His Ser Ala Pro Thr Gly Met Trp Glu Cys Pro Asp Phe
                245                 250                 255

Tyr Pro Val Ser Lys Gly Gly Ala Pro Arg Ala Gly Leu Glu Thr Ser
            260                 265                 270

Val Pro Pro Gly Pro Arg Val Lys His Val Leu Lys Asn Ser Leu Asp
        275                 280                 285

Leu Arg Arg Tyr Asp Tyr Tyr Thr Val Gly Thr Tyr His Pro Arg Ala
    290                 295                 300

Glu Arg Tyr Val Pro Asp Asp Pro Ala Gly Asp Glu His Arg Leu Arg
305                 310                 315                 320

Tyr Asp Tyr Gly Asn Phe Tyr Ala Ser Lys Thr Phe Tyr Asp Pro Ala
                325                 330                 335

Lys Arg Arg Arg Ile Leu Trp Gly Trp Ala Asn Glu Ser Asp Thr Ala
            340                 345                 350

Ala Asp Asp Val Ala Lys Gly Trp Ala Gly Ile Gln Ala Ile Pro Arg
        355                 360                 365

Thr Val Trp Leu Asp Pro Ser Gly Lys Gln Leu Leu Gln Trp Pro Ile
    370                 375                 380

Glu Glu Val Glu Ala Leu Arg Ala Lys Ser Val Thr Leu Arg Asn Arg
385                 390                 395                 400

Val Ile Lys Ala Gly His His Val Glu Val Thr Gly Ile Gln Thr Ala
                405                 410                 415

Gln Ala Asp Val Glu Val Ser Phe Glu Val Ser Pro Ala Ala Leu Ala
```

420 425 430

Gly Ala Glu Thr Leu Asp Pro Ala Leu Ala Tyr Asp Ala Glu Arg Leu
435 440 445

Cys Gly Val Lys Arg Ala Asp Val Arg Gly Gly Val Gly Pro Phe Gly
450 455 460

Leu Trp Val Leu Ala Ser Ala Asn Arg Lys Glu Arg Thr Ala Val Phe
465 470 475 480

Phe Arg Val Phe Lys Pro Ala Ala Gly Asp Lys Pro Val Val Leu Met
485 490 495

Cys Thr Asp Pro Thr Lys Ser Ser Leu Asn Pro Asn Leu Tyr Arg Pro
500 505 510

Thr Phe Ala Gly Phe Val Asp Thr Asp Ile Ser Asn Gly Lys Ile Ser
515 520 525

Leu Arg Ser Leu Ile Asp Arg Ser Val Val Glu Ser Phe Gly Ala Gly
530 535 540

Gly Lys Thr Cys Ile Leu Ser Arg Val Tyr Pro Ser Leu Ala Ile Gly
545 550 555 560

Lys Asp Ala Arg Leu Tyr Val Phe Asn Asn Gly Arg Ala His Val Lys
565 570 575

Val Ser Arg Leu Thr Ala Trp Glu Met Lys Lys Pro Val Met Met Asn
580 585 590

Gly Ala

<210> 3
<211> 4480
<212> DNA
<213> Artificial

<220>
<223> Construct pBETL-9 - Incw2

<220>
<221> promoter
<222> (1)..(1910)
<223> promoter pBETL9

<220>
<221> gene
<222> (1992)..(3773)
<223> Incw2 coding sequence

```
<220>
<221>  misc_feature
<222>  (3774)..(3776)
<223>  Stop signal

<400>  3
tcgagttact catgatggtc atctaggatc atagaccatc cccacagacc aacatgagtc      60

ttttctacgc actttgttca ctcgtgtgca tcaaagaaaa cttcttggtt ggtcactcat     120

ccaaaaattg ctctgagcca agcatgctta tcttagaggt ttttttgaga taagcttctg     180

aaaaagaagg tgcaccttgt ttgtatgagt attataccat tcctattaag ccttggacaa     240

agatatcaca atccacctag gccaagatat cacatttccc aacttagtct ataaaaggac     300

tagacaagac atctccttag aagagaagcc ctacctcttg tgcccataac aggcacctcc     360

aacttgagaa ctaatttcac aaagagtcac gctcttggga actccatgta ctcatatgta     420

cacactacat ctaatgcata gaaacaccaa gatcacattg tactagcaaa atgtcataga     480

agactagtta aaaccttgtt tggtctgctc aaacttaaca aatcacctag gagacatgct     540

agaagtatct caacagagaa tgacaccata tagtagtggc accaagtgcc taatctgcac     600

acaaaaaaat cgtaccatac atgacatcaa ggcttaataa tagagtgtat gttaaagcga     660

gcatgcaacc tatgagtggt atgtaggagt taggtttaaa caaggtaatg gctcaagcac     720

cacacatcct accacaatgt cgtaataaat ataaaagcac tagcaatcta tttagcatgc     780

ctaaatggga tactatgagg ttgggtggga tgtggcacct ttgtataatg gcccagttcc     840

ttagtgtagt cttgatcctc cccgttatgt tgagactcct ctagggattt tgtaggaatc     900

atcaaatttt cataagcaat ttcttgtgca caaagaacca aatagattga aaaagtttca     960

aattcactca aacacaaaac catggcacat agcttatgtg acaaaatatt tgggacacta    1020

gtttcatatt ttttgagatc atataagttt attatcaaac tcccaaggat taaattattt    1080

tttgaaaaaa aagaaaaaag ggaaaacatc ataaggtgac acatggcaac ctctgaatga    1140

ctagactttt accatctctc aggtgggtct ggtcaacaat cactgttggt cggtccttac    1200

cttgcctaga cgggtcctta gtaggcctac tgggttgagt tatgggataa attgtggcct    1260

agaaacatac cagtccacca accttgggac cacttaaaaa attgcatctt tcaccattat    1320

actatttaga tgtttttaaa aaacaatcat aacttttaca tcgaaatcaa aactagacaa    1380

attttatact ttcacagagc agcagaaatt tatacaatat gattgaatac aagatgtagg    1440

acccaatgga gagaattttt tgtctcctat atgcttgaat acccaacata atatcttcgc    1500

aacatactat ctatctaata gaaaaattat aatatagtta aatacttaag tagtatctag    1560

tggatagaat tcaatatctc ctacatgcat gaggagtaat atctactaga catgcaacat    1620

atttttatct atctaataga atatatataa taaagttaaa tattatatgc atcacctact    1680

atatataatt tgatatcttt tagatgtata agggactaag aataatatct ctagcacaca    1740
```

```
tgcaatgcat tatctatcta aatatattat ataatagtta aatattaatt atacgtagtc   1800
taaacctaca tataagccta cccatcccca cttaaagatc tcagtgtcac acatagacca   1860
tacatctcac ttcgccaaaa aaattccgtc aacagttgaa gttataccct ctagagcttg   1920
atatcacaag tttgtacaaa aaagcaggct ccgcggccgc ccccttgaat tcggcacgag   1980
gagtgaggcc aatgagagcc ctcgtagtcg taagtttcgc ttcggcgtgc ttgctgctgc   2040
tgttgcagct cgcaggagcg tcgcatgtcg tctacaacta caaggacctc gaagccgagg   2100
ctgctgcggc gacggaccag gtgccgccgt ccatcgtcaa ccccctgctc aggacggggt   2160
accacttcca gcccccaag aactggatca atgatcccaa cgcgcccatg tactacaagg   2220
ggtggtacca tttcttctac caatacaatc ccaagggcgc cgtatggggc aacatcgtgt   2280
gggcgcactc ggtgtcgcga gacctcatca actgggtggc gctagagccg gcgctcagac   2340
caagcatccc gggcgacagg tacggctgct ggtccgggtc ggcgacggtg ctgcccgacg   2400
gcggcggccc ggtgatcatg tacacgggcg tggaccaccc ggacatcaac taccaggtcc   2460
agaacgtggc gtacccgaag aacgtgtcgg acccactgct ccgggagtgg gtgaagccct   2520
cgcacaaccc cgtcatcgtc cccgagggcg gcatcaacgc gacgcagttc cgcgacccga   2580
ccacggcctg gcgcggcccc ggccacgagc agtggcggct gctcgtcggc agcgcggcgg   2640
ggtcgtcgcc gccccgcggc gtggcgtacg tgtaccgtag ccgcgacttc cggcggtgga   2700
ggcgggtgca gcggccgctg cactcggcgc ccacggggat gtgggagtgc cctgacttct   2760
acccggtgag caagggcggc gcgccgcgcg cggggctcga gacgtccgtg ccgcccggcc   2820
caagggtcaa gcacgtgctc aagaacagcc tcgacctccg gcggtacgac tactacaccg   2880
tgggcacgta ccacccgagg gccgagcggt acgtgccgga cgaccccgcc ggcgacgagc   2940
accgcctgcg ctacgactac ggcaacttct acgcgtccaa gacgttctac gacccggcca   3000
agcggcgccg catcctgtgg ggctgggcta acgagtccga caccgccgcc gacgatgtgg   3060
ccaaaggctg ggctggaatc caggcgattc caaggacggt gtggctggac cccagcggga   3120
agcagctgct gcagtggcct atcgaggagg tggaggcgct gagagcaaag tcggtcactc   3180
tcaggaacag ggtaatcaag gcaggacatc acgtcgaggt gaccgggata caaacggcac   3240
aggctgacgt ggaggtgagc ttcgaagtgt cgccggcggc cctggccggt gccgagacgc   3300
tggacccggc gctggcctac gacgcggaga ggctgtgcgg cgtgaagcgc gcggacgtga   3360
ggggcggcgt ggggccgttc gggctgtggg tgctggcctc cgccaaccgc aaggagagaa   3420
ccgcggtgtt cttcagggtg ttcaagcccg ccgccggcga caagcccgtg gtgctcatgt   3480
gcaccgaccc taccaagtcg tccctgaacc cgaacctgta ccgcccgaca tttgcaggat   3540
ttgtcgacac ggacatctcg aacggcaaga tatccctgag aagcctgatc gaccggtcgg   3600
tcgttgagag cttcggagcc gggggcaaga cctgcatcct ctccagggtc tacccgtcgc   3660
tggccatcgg caaggacgct cgcctctacg tgttcaacaa cgggagggcg cacgtcaagg   3720
```

tgtcccgtct caccgcgtgg gagatgaaga agccggtcat gatgaacggg gcctgaagat 3780

cttgaataaa tatatcactc atgcatgaca tatagtggcg cctgagaaat ctttccttat 3840

atatatatat attttagata tatcgtttaa gaattcatag cgctaagtcg acatcccggg 3900

ttctgcaggg tgaagggtgg gcgcgccgac ccagctttct tgtacaaagt ggtgatatcc 3960

taaatgctct taactgagct aattatgtaa tgcacataca catatttaca tagatatgca 4020

tatttatata tagcatgtat attgtactac atgcattgct tcttaataca tgtagtaaag 4080

atatatgcaa aaatagtcga aagatttgtt tacatataaa atcaccaata tttattgtta 4140

ttgtattttc atgaataaag taataagatt atttgtctaa tattttgatt tactagtact 4200

agaaatgaaa aggaatatgc acaatttcag cattatagtt tggtaggcaa aatggagtga 4260

gaatagagtt tcatagtata tactaaggtt cttaattgtg caaatagttg atacaagtca 4320

catgggccaa gtttgtaaat cttaaatcga aatatgcctt cttctttttt tgcatgaaaa 4380

tgctagtaat ttataagtgt gtttttcaat aagagatgct aaataccaaa attaacctag 4440

ttttcagtga gcgcttgcat tattgtggca tgtttaaact 4480

<210> 4
<211> 1785
<212> DNA
<213> Zea mays

<400> 4
atgagagccc tcgtagtcgt aagtttcgct tcggcgtgct tgctgctgct gttgcagctc 60

gcaggagcgt cgcatgtcgt ctacaactac aaggacctcg aagccgaggc tgctgcggcg 120

acggaccagg tgccgccgtc catcgtcaac cccctgctca ggacggggta ccacttccag 180

ccccccaaga actggatcaa tgatcccaac gcgcccatgt actacaaggg gtggtaccat 240

ttcttctacc aatacaatcc caagggcgcc gtatggggca acatcgtgtg ggcgcactcg 300

gtgtcgcgag acctcatcaa ctgggtggcg ctagagccgg cgctcagacc aagcatcccg 360

ggcgacaggt acggctgctg gtccgggtcg gcgacggtgc tgcccgacgg cggcggcccg 420

gtgatcatgt acacgggcgt ggaccacccg gacatcaact accaggtcca gaacgtggcg 480

tacccgaaga acgtgtcgga cccactgctc cgggagtggg tgaagccctc gcacaacccc 540

gtcatcgtcc ccgagggcgg catcaacgcg acgcagttcc gcgacccgac cacggcctgg 600

cgcggccccg gccacgagca gtggcggctg ctcgtcggca gcgcggcggg gtcgtcgccg 660

ccccgcggcg tggcgtacgt gtaccgtagc cgcgacttcc ggcggtggag gcgggtgcag 720

cggccgctgc actcggcgcc cacggggatg tgggagtgcc ctgacttcta cccggtgagc 780

aagggcggcg cgccgcgcgc ggggctcgag acgtccgtgc cgcccggccc aagggtcaag 840

cacgtgctca agaacagcct cgacctccgg cggtacgact actacaccgt gggcacgtac 900

cacccgaggg ccgagcggta cgtgccggac gaccccgccg gcgacgagca ccgcctgcgc 960

```
tacgactacg gcaacttcta cgcgtccaag acgttctacg acccggccaa gcggcgccgc   1020
atcctgtggg gctgggctaa cgagtccgac accgccgccg acgatgtggc caaaggctgg   1080
gctggaatcc aggcgattcc aaggacggtg tggctggacc ccagcgggaa gcagctgctg   1140
cagtggccta tcgaggaggt ggaggcgctg agagcaaagt cggtcactct caggaacagg   1200
gtaatcaagg caggacatca cgtcgaggtg accgggatac aaacggcaca ggctgacgtg   1260
gaggtgagct tcgaagtgtc gccggcggcc ctggccggtg ccgagacgct ggacccggcg   1320
ctggcctacg acgcggagag gctgtgcggc gtgaagcgcg cggacgtgag gggcggcgtg   1380
gggccgttcg ggctgtgggt gctggcctcc gccaaccgca aggagagaac cgcggtgttc   1440
ttcagggtgt tcaagcccgc cgccggcgac aagcccgtgg tgctcatgtg caccgaccct   1500
accaagtcgt ccctgaaccc gaacctgtac cgcccgacat ttgcaggatt tgtcgacacg   1560
gacatctcga acggcaagat atccctgaga agcctgatcg accggtcggt cgttgagagc   1620
ttcggagccg ggggcaagac ctgcatcctc tccagggtct acccgtcgct ggccatcggc   1680
aaggacgctc gcctctacgt gttcaacaac gggagggcgc acgtcaaggt gtcccgtctc   1740
accgcgtggg agatgaagaa gccggtcatg atgaacgggg cctga                   1785
```

```
<210>  5
<211>  30
<212>  DNA
<213>  Artificial

<220>
<223>  pBETL9forXho

<400>  5
ccctcgagtt actcatgatg gtcatctagg                                      30


<210>  6
<211>  32
<212>  DNA
<213>  Artificial

<220>
<223>  pBETL9revXba

<400>  6
gctctagagg gtataacttc aactgttgac gg                                   32
```

## Description

The invention relates to the field of plant improvement, in particular of the improvement of yield for plants.

In the context of the present invention a cereal shall mean in particular maize, rice, wheat, barley, sorghum, millet, oats, rye, triticale (hybrid of wheat and rye), fonio, as well as two pseudocereals, namely buckwheat and quinoa.

Maize and wheat are the preferred cereals according to the invention.

In agriculture, yield is the amount of product harvested from a given acreage (eg weight of seeds per unit area). It is often expressed in metric quintals (1 q = 100 kg) per hectare in the case of cereals. It is becoming increasingly important to improve the yield of seed crops to feed an expanding population and, more recently, for biofuel production. One strategy to increase the yield is to increase the seed size, provided that there is not a concomitant decrease in seed number.

The invention provides a construct which can be used as a transgene for obtaining transgenic plants that have an increased yield with regards to isogenic plants that do not contain said transgene. In particular, the purpose is to have an increased yield, for the transgenic plants, in stressed conditions and in particular in hydric stress conditions (drought), or in heat stress conditions.

As intended herein, two plants are said to be "isogenic" when they differ at very few loci (less than 20, more preferably less than 10), and when one does carry the transgene, while the other does not. These plants can also be called "virtually isogenic".

The endosperm, which forms most of the volume and weight of the kernel, can be divided into three parts: starchy endosperm, aleurone layer and basal transfer layer.

The role of the starchy endosperm is to provide the nutrients to the seed. It is thus mainly composed by cells filled with reserves, such as starch granules, or protein bodies. These cells die during dessication of the seed and this compartment is thus composed of dead cells in mature seeds.

The aleurone layer is a one-celled layer of cells in contact with the pericarp. Basically, these cells accumulate lipids and storage proteins. The cells remain active until maturation and play an important role during germination.

The basal endosperm transfer layer (BETL) cells are in contact with the pedicel at the base of the endosperm. They transport the nutrients from the mother plant to the embryo surrounding region, as direct transport is not possible since vascular tissue does not extend beyond the pedicel.

Shen et al (Maydica 57, 147-153; 2012) described that overexpression of an Incw2 gene in endosperm improved yield related traits in maize. They used the 27 kD zein promoter to drive expression of this gene. Said promoter is expressed throughout the endosperm by 10 and 15 DAP (days after pollination), and RNA transcript were detected throughout most regions of the starchy endosperm by 15 DAP, except in the aleurone and basal transfer cells as indicated by Woo et al (The Plant Cell, Vol. 13, 2297-2317, 2001). Although the authors observed increase in some yield-related traits, they didn't demonstrate that yield was actually increased. In fact, it is known that some yield related traits may be increased without any increase in yield: in particular, Hughes et al (Plant Biotechnol J. 2008 Oct;6(8):758-69. Epub 2008 Jul 8) describe that, although *arf2* mutants have larger seeds than wild, the yield is lower due to the production of fewer seeds per plant (Figure 3c). It is thus not possible to conclude that it is possible to increase yield, in particular in stressed conditions, from the teachings of Shen *et* al.

The invention relates to a nucleic acid construct comprising: a) a promoter active in the BETL, operatively linked to b) a nucleic acid coding for a IncW2 protein, an allele of which is represented by SEQ ID N° 2 (the cDNA is represented by SEQ ID N° 4).

It is to be noted that the protein IncW2 is not naturally expressed in the BETL. Consequently, the nucleic acid construct is not found in nature.

It is also to be noted that SEQ ID N° 2 represents and exemplifies an allele of the IncW2 protein in maize. There exists different other alleles of this protein, which all possess the same activity than this protein, and can be used in the above-identified nucleic acid construct. One can cite, as such alleles, the proteins that are disclosed in GenBank with the following accession numbers: NP_001105596.1, AAF06992.1, AAF06991.1, AAD02510.1, ACG47298.1. This list is not exhaustive, and other IncW2 proteins can also be used in said nucleic acid construct. These may be identified, by applying the BLASTP program (especially the BLASTP 2.2.29 program) (Altschul et al, (1997), Nucleic Acids Res. 25:3389-3402; Altschul et al, (2005) FEBS J. 272:5101-5109) to SEQ ID N° 2, using the following algorithm parameters:

Expected threshold : 10
Word size : 3
Max matches in a query range: 0
Matrix: BLOSUM62
Gap Costs: Existence 11, Extension 1.
Compositional adjustments: Conditional compositional score matrix adjustment
No filter for low complexity regions

The proteins that can be used in the context of the above construct are preferably the ones that present a Max score above 1000. They would also preferably present an identity (as indicated by the BLAST2P software) equal or above 90%, preferably, equal or above 95% more preferably equal or above 97% more preferably equal or above 98%, more preferably equal or above 99%.

In a specific embodiment, said promoter active in the BETL is not an imprinted promoter.

Some promoters active in the BETL promoters have been described in WO1999050427 and in Hueros (Plant Cell, Vol. 7, 747-757, 1995). These promoters are predominantly functional in the endosperm and particularly in the BETL and preferably exclusively in the BETL.

A promoter "active in the BETL" of a plant is a promoter that allows expression of a nucleic acid sequence operatively linked to it in the BETL of said plant. In a specific embodiment, said promoter is active in the BETL at least during the days following pollination, in particular between 0 and 15 DAP (days after pollination). In another embodiment, said promoter is active in the BETL at least between 0 and 24 DAP. In another embodiment, said promoter is active in the BETL at least between 8 and 24 DAP

In another embodiment, said promoter is predominantly functional in the BETL, *i.e.* said promoter can be active in other tissues than the BETL, but the principal expression of a nucleic acid sequence encoding a protein operatively linked to it is in the BETL. This can be verified, using various techniques known by the person skilled in the art, such as quantification of the RNA expression of said nucleic acid sequence in various tissues by Northern blot, or of the protein expression in various tissues by Western blot.

A promoter exclusively expressed in the BETL is active exclusively in the BETL, *i.e.* it is not possible to detect expression of a nucleic acid sequence encoding a protein operatively linked to it in other tissues than the BETL.

In a specific embodiment, said promoter active in the BETL is the BETL9 promoter, the sequence of which is SEQ ID N° 1.

It is to be noted that, although SEQ ID N° 1 is herein used to define BETL9, a sequence that is at least 90 % identical, preferably at least 95 % identical, more preferably at least 96 % identical, more preferably at least 97 % identical, more preferably at least 98 % identical, more preferably at least 99 % identical, more preferably at least 99.5 % identical, more preferably at least 99.7 % identical, more preferably at least 99.9 % identical, more preferably at least 99.95 % identical to SEQ ID N° 1 can also be considered as being a BETL9 promoter. This is because it is known that it is possible to change or delete (especially in the 5' end) a few nucleic acids in a promoter without modifying its pattern of expression. Comparing the patterns of expression of a modified promoter and of the promoter depicted by SEQ ID N° 1 can be performed using any reporter gene known in the art, such as the luciferase, GUS or GFP genes.

In a specific embodiment, the invention thus relates to a nucleic acid construct (also referred to as an expression cassette) comprising a nucleic acid molecule comprising the BETL9 promoter represented by SEQ ID N° 1, operatively linked to a nucleic acid coding for an INCW2 protein.

The BETL9 promoter is also described as sequence 18 from US20090307795, sequence 32 from US20080313778 and sequence 69 from US20120011621.

In particular the nucleic acid construct comprises SEQ ID N° 3. In another embodiment, the nucleic acid constructs comprises the sequence between nucleotides 1 and 3773 (or 1 and 3776) of SEQ ID N° 3.

The invention also encompasses a vector containing the expression cassette of the invention.

A vector, such as a plasmid, can thus be used for transforming host cells. The construction of vectors for transformation of host cells is within the capability of one skilled in the art following standard techniques.

The decision as to whether to use a vector for transforming a cell, or which vector to use, is guided by the method of transformation selected, and by the host cell selected.

Where a naked nucleic acid introduction method is used, then the vector can be the minimal nucleic acid sequences necessary to confer the desired phenotype, without the need for additional sequences.

Possible vectors include the Ti plasmid vectors, shuttle vectors designed merely to maximally yield high numbers of copies, episomal vectors containing minimal sequences necessary for ultimate replication once transformation has occured, transposon vectors, including the possibility of RNA forms of the gene sequences. The selection of vectors and methods to construct them are commonly known to persons of ordinary skill in the art and are described in general technical references (Mullis, K B (1987), Methods in Enzymology).

For other transformation methods requiring a vector, selection of an appropriate vector is relatively simple, as the constraints are minimal. The apparent minimal traits of the vector are that the desired nucleic acid sequence be introduced in a relatively intact state. Thus, any vector which produces a plant carrying the introduced DNA sequence should be sufficient. Also, any vector which introduces a substantially intact RNA which can ultimately be converted into a stably maintained DNA sequence should be acceptable.

For transformation methods within a plant cell, one can cite methods of direct transfer of genes such as direct micro-injection into plant embryos, vacuum infiltration or electroporation, direct precipitation by means of PEG or the bombardment by gun of particules covered with the plasmidic DNA of interest.

It is preferred to transform the plant cell with a bacterial strain, in particular *Agrobacterium,* in particular *Agro-*

*bacterium tumefaciens.* In particular, it is possible to use the method described by Ishida et al. (Nature Biotechnology, 14, 745-750, 1996) for the transformation of Monocotyledons.

However, any additional attached vector sequences which confer resistance to degradation of the nucleic acid fragment to be introduced, which assists in the process of genomic integration or provides a means to easily select for those cells or plants which are actually, in fact, transformed are advantageous and greatly decrease the difficulty of selecting useable transgenic plants.

The vector can exist, for example, in the form of a phage, a plasmid or a cosmid. The construction of such expression vectors for transformation is well known in the art and uses standard techniques. Mention may be made of the methods described by Sambrook *et al.* (1989).

For transforming bacteria, a vector is generally defined as being a nucleic acid molecule that possesses elements that allows it to be maintained within said host cell (such as an origin of replication that works in this bacterial host cell).

The invention also encompasses a host cell containing the expression cassette as described above.

The decision as to whether to use a given host cell, or which host cell to use, is guided by the method of transformation.

The host cell can be any prokaryotic or eukaryotic cell. Any of a large number of available and well-known host cells may be used in the practice of this invention. The selection of a particular host is dependent upon a number of factors recognized by the art. These include, for example, compatibility with the chosen expression vector, bio-safety and costs. Useful hosts include bacteria such as *E.* coli sp. or *Agrobacterium.* A plant host cell, may be also used, notably an Angiosperm plant cell, Monocotyledon as Dicotyledon plant cell, particularly a cereal or oily plant cell, selected in particular from the group consisting of maize, wheat, barley, rice, rape and sunflower, preferentially maize.

More particularly, the host cell used in carrying out the invention is *Agrobacterium tumefaciens,* according to the method described in the article of An *et al.,* 1986, or *Agrobacterium rhizogenes,* according to the method described in the article of Jouanin *et* al., 1987.

In a specific embodiment, said expression cassette is stably integrated within the genome of said host cell. This embodiment is particularly interesting for plant host cells. Stable integration within the genome means that the expression cassette can be transmitted to the progeny of said host cell upon division.

The invention also encompasses a plant containing at least one cell containing the expression cassette as defined above, preferably stably integrated within its genome.

A part of a transgenic plant, in particular fruit, seed, grain or pollen, comprising such a cell or generated from such a cell is also encompassed by the invention.

It is reminded that a whole plant can be regenerated from a single transformed plant cell. Thus, in a further aspect the present invention provides transgenic plants (or parts of them) including the expression cassette according to the invention. The regeneration can proceed by known methods.

The seeds which grow by fertilization from this plant, also contain this transgene in their genome.

Said plant or part of a plant according to the invention can be a plant or a part of it from various species, notably an Angiosperm, Monocotyledons as Dicotyledons.

It is preferably a cereal or oily plant. As used herein, the term "oily plant" denotes a plant that is capable of producing oil, and preferably that is cultivated for oil production.

Said plant is preferably selected from the group consisting of maize, rice, wheat, barley, rape and sunflower. In a preferred embodiment, said plant is maize. In another preferred embodiment, said plant is wheat.

The invention thus relates in particular to a transgenic maize or a transgenic wheat, containing at least one cell comprising, stably integrated in its genome, the expression cassette of the invention.

In a specific embodiment, said plant, in particular said maize, comprises multiple cells containing, stably integrated in their genome, the expression cassette of the invention. In this embodiment, it is possible that some cells of said plant do not contain the transgene.

Due to the use of the BETL9 promoter, expression of the INCW2 protein is thus predominant in the endosperm, and in particular in the BETL, preferably an exclusive pattern expression in the BETL. This may be observed by performing Northern blot on RNA obtained from different organs of the plant, and detecting an expression at least ten times higher in the endosperm, and in particular in the BETL, than in other organs.

In another embodiment, said transgene (comprising the expression cassette of the invention) is present in all cells of said plant, in particular said maize or wheat.

In another embodiment, the transgene is introduced within the plant cells such as being expressed transiently, or through a genetic construct not integrated in the genome. Thus, agro-infiltration or any other methods, such as injection or spray, are contemplated for transient expression.

Hybrid plants obtained by crossing plants according to the invention also form part of the invention, when they contain at least one cell containing the expression cassette of the invention.

Any plant as described above can contain one or more transgenes in addition to the cassette according to the invention. One may mention transgenes conferring male sterility, male fertility, resistance to a herbicide (notably glyphosate, glufosinate, imidazolinone, sulfonylurea, L-phosphinotricine, triazine, benzonitrile), resistance to insects (notably a transgene coding for a *Bacillus thuringiensis* toxin), tolerance to water stress. These plants can be obtained by crossing said plants of the invention with other plants containing said transgenes. Alternatively, plants can be co-transformed with an expression cassette containing several different transgenes, including the transgene of the invention.

As demonstrated in the examples, said transgenic plants comprising an expression cassette according to the invention present an increased yield as compared to control plants corresponding to non-transgenic plants not comprising said expression cassette.

Said increased yield may be observed in normal conditions or in stress conditions.

Increased yield in stress conditions (or stress tolerance) can be measured by the ability of the transgenic plant to maintain yield under stress conditions compared to normal conditions (which is considered to be achieved when the yield observed in stressed conditions is at least 90%, 94%, 95%, 96%, 97%, 98%, 99% or 100% of the yield obtained for the same plant in non-stressed (normal) conditions). It can also be measured by the ability of the transgenic plant to increase yield under stress conditions compared to control plants grown under stress conditions (at least 101%, 102%, 103%, 104%, 105%, 106%, 107%, 108%, 109%, 110%, 111%, 112%, 113%, 114%, 115%).

As intended herein, stress conditions comprise specific conditions that are applied to a plant at a specific development stage such as that they induce a yield decrease of at least 8%, 10%, preferentially at least 15% and more preferentially at least 20% between the control plants in normal and in stress conditions. As a matter of illustration, one can cite heat stress conditions that may be applied during the flowering stage (in particular for wheat) or hydric stress before the flowering stage or after the fertilization, (in particular during the grain filling stage for maize).

Consequently, the invention also relates to various methods of using the plants of the invention.

Therefore, the invention also relates to a method for obtaining a transgenic plant containing at least one cell comprising a transgene comprising the expression cassette as described above, comprising the steps consisting of:

a) transforming at least a plant cell or plant tissue with a vector containing the nucleic acid construct according to the invention;
b) cultivating the cell(s) or plant tissue thus transformed so as to generate a plant containing in its genome at least the expression cassette of the invention,
whereby said generated plant contains at least one cell which comprises the transgene comprising the expression cassette as described above.

Said generated plant may present an increased yield, in normal or stressed conditions, as compared to an isogenic plant that does not contain said expression cassette in its genome, or shall be able to maintain the yield observed in normal conditions when grown in stressed conditions.

In this method, it is clear that the measure of yield is checked by sowing and harvesting of a multiplicity of plants that contain the transgene, the yield of which is then compared with the yield obtained with a second group of plants not containing said transgene, and this under the same culture conditions (sowing and harvest at the same time, on comparable parcels, use of the same amount of fertilizers, water...).

It is also clear that comparison is to be performed on a second group of plants that is isogenic to the plants having the transgene. As indicated above, these "isogenic" plants differs from the plants harboring the transgene at very few loci (less than 20, more preferably less than 10), in addition to not carry said transgene. In particular a plant carrying the transgene isogenic to another plant of interest may be obtained by at least four backcrosses in the isogenic plant of interest, followed by at least one self-fertilization. Preferably, the isogenic plants are homozygous lines.

Said generated plant can also be used in a selection (breeding) process for obtaining a plant with improved yield.

The invention thus also relates to a method for producing a plant that can be used in a selection (breeding) process or scheme for obtaining a plant with improved yield, comprising the step of transforming a plant cell with a vector according to the invention, and regenerating a transgenic plant which comprises at least one cell which contain the transgene comprising the expression cassette as described above.

The introgression of the transgene in a given plant is in particular carried out by selection, according to methods known in the art (crossing and self-pollination). The plants are in particular selected using molecular markers.

The principle is recalled below:

A series of back crosses are performed between the elite line (in which one wishes to introduce the determinant) and a line that already carries said determinant (the donor line). During the back crosses, one can select individuals carrying the determinant and having recombined the smallest fragment from the donor line around the determinant. Specifically, by virtue of molecular markers, the individuals having, for the markers closest to the determinant, the genotype of the elite line are selected.

In addition, it is also possible to accelerate the return to the elite parent by virtue of the molecular markers distributed over the entire genome. At each back cross, the individuals having the most fragments derived from the recurrent elite parent will be chosen.

Selection is important as it is often preferable to sow and harvest plant lines that have been optimized, in particular for the location in which they are cultured. Consequently, one needs to introduce the transgene in said adapted lines having otherwise agronomic quality characteristics.

The invention also relates to a method for obtaining a plant containing a transgene, wherein said transgene comprises the expression cassette as described above, comprising the steps of

a) Performing the method as described above (transformation of plant cells and regeneration) in order to obtain a transgenic plant, wherein said transgene comprises said expression cassette,
b) crossing said transgenic plant with a plant line which does not contain said transgene (the receiver plant line)
c) selecting, among the progeny, plants that contain said transgene and that have a good genome ratio with regard to said receiver plant line,
d) back-crossing said selected plants with said receiver plant line
e) repeating steps c) and d) if necessary until a line isogenic with said receiving line (and containing said transgene) is obtained,
f) optionally, performing self-fertilization in order to obtain a plant homozygotic for the transgene.

The selection of step c) is preferably performed, by genotyping using molecular markers (for example microsatellite markers), making it possible to define the contribution of each of the two parents to the progeny. One would thus select, in the progeny, plants carrying the transgene and having more markers from the receiver plant line than from the parent containing the transgene.

Plants (in particular maize or wheat) which possess the transgene, may also be selected from the progeny, in a conventional manner by molecular biology methods (such as PCR or Southern blotting).

The invention also relates to a method for growing a plant, comprising the step of sowing a plant seed, wherein said plant seed contains the nucleic acid construct as described above, and growing plants from this sowed seed.

The invention also relates to a method for increasing plant yield under normal conditions for plant harvest, comprising the step of sowing plant seeds, wherein said plant seeds contain the expression cassette of the invention and growing plants from these sowed seeds, and wherein the yield obtained from said grown plants is increased as compared to the yield obtained from isogenic plants grown from seeds which do not contain said expression cassette

The invention also relates to a method for increasing or maintaining plant yield under stressed conditions, comprising the step of sowing plant seeds, wherein said plant seeds contain the nucleic acid construct as described above, and growing plants from these sowed seeds, wherein the growing phase is made under stress conditions, and wherein the yield obtained from said grown plants is increased as compared to the yield obtained from plants grown from seeds which do not contain said nucleic acid construct or the yield obtained from said grown plants is maintained as compared to the yield obtained from plants containing said nucleic acid construct and grown in normal conditions.

The invention also relates to a method of growing plants, comprising the step of sowing seeds containing the nucleic acid construct as described above, , and growing plants from the sowed seeds.

The invention may also comprise the step of harvesting said plants.

The invention also relates to a method for harvesting plants comprising the step of harvesting plants of the invention.

In particular, in the methods as described above, a hydric stress is applied to the plants during their growth.

A method for selecting (screening, identifying) a plant that can be used in a selection (breeding) process for obtaining a plant with improved yield, which comprises the step of selecting, in a population of plants, the plants containing the expression cassette as described above, is also part of the invention.

A breeding process for obtaining a plant with improved yield is performed as follows: the yield of a plurality of plants gives the reference yield level which is to be improved. The plant with improved yield is obtained, when the yield observed after sowing and harvesting said plant is higher than the yield of reference. Said plant with improved yield is obtained by known methods in the art, by crossing, back-crossing and stabilizing plants which present a yield

In a specific embodiment, the selection is performed through the use of a marker that is specific to the transgene. In this embodiment, the selection step is thus preferably preceded by a step comprising genotyping said population of cereals.

In a specific embodiment, the selection step is preceded by a step comprising extracting the RNA from the individuals in said population.

In a specific embodiment, the selection step is preceded by a step comprising extracting proteins from the individuals in said population.

In a specific embodiment, said population is the progeny obtained from crossing a transgenic plant, wherein said transgene comprises the expression cassette as described above, with a plant line which does not contain said transgene (the receiver plant line).

A method for identifying a plant with improved yield, which comprises the step of identifying, in a population of plants, the plants containing the expression cassette as described above, is also part of the invention. Improved yield is determined after comparison with a isogenic plant which does not contain the expression cassette.

In a specific embodiment, the identification is performed through the use of a marker that is specific to the transgene. In this embodiment, the identification step is thus preferably preceded by a step comprising genotyping said population of cereals.

In a specific embodiment, the identification step is preceded by a step comprising extracting the RNA from the individuals in said population.

In a specific embodiment, the identification step is preceded by a step comprising extracting proteins from the individuals in said population.

In a specific embodiment, said population is the progeny obtained from crossing a transgenic plant, wherein said transgene comprises the expression cassette as described above, with a plant line which does not contain said transgene (the receiver plant line).

The invention also relates to a method for obtaining a hybrid plant, wherein said hybrid plant contains the expression cassette as described above stably integrated within its genome. Said method comprises the step of crossing a first homozygous line, which contains said expression cassette stably integrated within its genome, with a second homozygous line.

This plant can be homozygous (if each homozygous parent has the expression cassette as described above stably integrated within its genome) or heterozygous for the transgene present on said expression cassette.

In a preferred embodiment, the methods are applied to a cereal (in particular, rice, maize, wheat, barley). It is preferred when said plant is maize or wheat.

It is also to be understood that the teachings of the invention make it possible to use sequences that present identity to SEQ ID N° 1 or SEQ ID N° 2 (preferably more than 95, 97, 98 or 99 % identity). Using these similar sequences to produce transgenic plants makes it possible to obtain the same technical effect: increase of yield for these transgenic plants (as compared with isogenic plants not carrying the transgene), in particular in stressed conditions, and specifically in hydric stress conditions. The degree of identity is defined by comparison with the entire sequence of reference. This may be performed using a sequence analysis software, such as the blast program. Using the sequences similarities and such softwares and databases, it is thus possible to identify the orthologs of Incw2 for other cereal plants than maize, that can be used in the invention (as a metter of illustration, one can cite the protein accessible under accession number ABM65157.1, from Sorghum bicolor, or the protein having accession number BAM74037.1 from Triticum aestivum, or the protein having accession numbers Q011S7.2 or NP_001052748.1 from Oryza Sativa, which are all cell wall invertases identified through the blastp program).

Preferably, the percentage of identity of two polypeptides is obtained by performing a blastp analysis with the sequence encoded by the nucleic acid according to the invention, and SEQ ID N° 2, using the BLOSUM62 matrix, with gap costs of 11 (existence) and 1 (extension).

Similar nucleotide sequences are aligned in order to obtain the maximum degree of homology (i.e. identity). To this end, it may be necessary to artificially introduce gaps in the sequence. Once the optimum alignment has been achieved, the degree of homology (i.e. identity) is established by recording all the positions for which the nucleotides of the two compared sequences are identical, with respect to the total number of positions. It may be obtained using the blastn software, with the default parameters as found on the NCBI web site.

## DESCRIPTION OF THE FIGURE

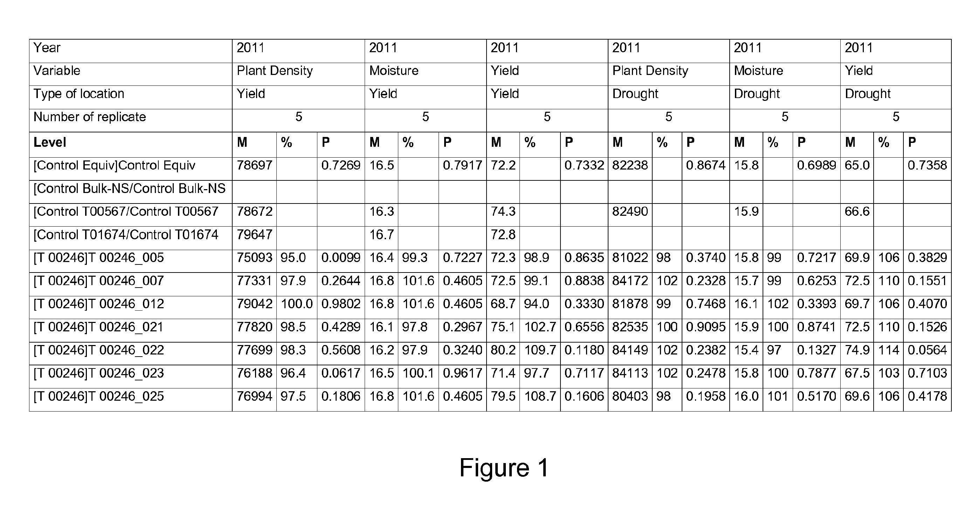

Figure 1 represents Table I, and compares the plant density, Moisture and Yield for transgenic plants exemplifying the invention, and control plants. Yield is expressed in (Qx/ha). In said figure, "M" means "Mean square", "%" means "% as compared to the control group", "P" means "P-value as compared to the control group". The results obtained from two locations ("Yield" and "Drought") are indicated. Location "Yield" corresponds to normal conditions, whereas location "Drought" corresponds to stressed conditions (deficit of water). The tested events are the plants

## EXAMPLES

### Example 1 - Cloning of ZmINCW2 downstream the BETL9 promoter and transformation

The ZmINCW2 coding sequence was cloned as an EcoRI fragment from anIncW2 cDNA into EcoR1--EcoRI-cut pBIOS503 forming the GATEWAY ENTRY clone pBIOS713. (pBIOS503 is a derivative of pENTRD/Topo (Invitrogen) containing a polylinker with the sites EcoRI - SalI- Sma I- Pst I between the aatL1 and aatL2 recombinase sites.)

The BETL9 gene was isolated by Hueros et al (1995). The promoter sequence is, in part, described as pEND1 Patent WO 00/12733. Figure 2 shows that by Northern and in situ analysis BETL9 is expressed in the lower half of the seed in the BETL.

The 1904bp maize BETL9 promoter was amplified by PCR from genomic DNA of the inbred line F2 using the primers:-

pBETL9forXho 5' CCCTCGAGTTACTCATGATGGTCATCTAGG 3' (SEQ ID N° 5),
and pBETL9revXba 5' GCTCTAGAGGGTATAACTTCAACTGTTGACGG 3' (SEQ ID N° 6).

These primers introduce an XhoI and an XbaI site 5' and 3' to the BETL9 promoter.

The PCR fragment was cloned as an XhoI, XbaI fragment into XhoI, XbaI-cut pBSKII forming pBETL9-BS. A GATEWAY cassette and a Sac66 polyadenylation sequence was cloned from pBIOS652 as a HindIII (filled), SacI fragment into XbaI (filled), SacI-cut pBETL9-BS thus forming pBIOS710. An LR clonase reaction was performed between pBIOS713 and pBIOS710 thus forming pBIOS740. The pBETL9-ZmINCW2-Sac66 polyA chimeric gene from pBIOS740 was cloned as a partial XhoI fragment into XhoI-cut pBIOS340, forming pBIOS749. (The binary vector pBIOS340 is a derivative of pSB12 (Komari et al.(1996)) containing a pActin+actin intron-NptII nos polyA chimeric gene for selection of maize transformants).

pBIOS749 was transferred into agrobacteria LBA4404 (pSB1) according to Komari et al (1996). Maize cultivar A188 was transformed with these agrobacterial strains essentially as described by Ishida et al (1996).

Analysis of the pBETL9-INCW2 transformed corn plants indicated that some plants overexpressed ZmINCW2.

**<u>Example 2 - Corn field trials</u>**

Field trials show that seed yield and the stability of yield is improved.

*<u>A - Field trials</u>*

*<u>Tested hybrids</u>*

Hybrids with a tester line were obtained from T3 plants issued from the INCW2 transgenic maize line (proBETL9 + ZmINCW2 + Sac66 term) chosen according to Example 1.

The transformants (T0) plant was first crossed with the A188 line thereby producing T1 plants. T1 plants were then self-pollinated twice, producing T3 plants which are homozygous lines containing the transgene. These T3 plants were then crossed with the tester line thereby leading to a hybrid. This hybrid is at a T4 level with regards to the transformation step and is heterozygous for the transgene. These hybrid plants are used in field experiments.

*<u>Control hybrids</u>*

Control hybrids are obtained as follows: *Control Equiv* corresponds to a cross between theA188 line (the line used for transformation) and the tester line.

Control T 00567 or T 01674 correspond to a cross between a null segregant (isolated after the second self-pollination of the T1 plants) and the tester line. Said null segregant is a homozygous line which does not bear the transgene. Although the null segregant theoretically presents the same genome as A188, it has undergone *in vitro* culture (via the steps of callus differentiation and regeneration) and may thus present mutations (either genetic or epigenetic) with regards to a A188 line that has not undergone in vitro culture.

These two control lines are used to avoid any effect that could be due to mutations (genetic or epigenetic) coming from *in vitro* culture.

*<u>Calculation of yield</u>*

Yield was calculated as follows:

During harvest, grain weight and grain moisture are measured using on-board equipment on the combine harvester.

Grain weight is then normalized to moisture at 15 %, using the following formula:

Normalized grain weight = measured grain weight x (100 - measured moisture (as a percentage)) / 85 (which is 100 - normalized moisture at 15 %).

As an example, if the measured grain moisture is 25 %, the normalized grain weight will be: normalized grain weight = measured grain weight x 75 / 85.

Yield is then expressed in a conventional unit (such as quintal per hectare).

*B - Experimental design*

Field trials were conducted in 2011 on different locations. Plants were sown between June 15 and June 25 2011. Harvest was between November 10 and November 12, 2011.

The experimental block comprises 5 replicates. The experimental design was Randomized complete block or Lattice. Each replicate comprised of two row plots with about 62 plants per plot at a density of 73 800 plants/ha.

Controls were used present in this experiment as described above (null segregant T00567 or T 01674 and a control equivalent (A188 crossed with the tester line).

Results are represented in Table I, with the yield expressed in (Qx/ha).

In said table, "M" means "Mean square", "%" means "% as compared to the control group", "P" means "P-value as compared to the control group".

This table demonstrates that the transgenic plants present an increased yield (normalized for moisture) in normal or stress conditions (drought). No other difference of phenotypes were observed for these plants compared to the control plant

## Claims

1. A nucleic acid construct comprising: a) a promoter active in the BETL, operatively linked to b) a nucleic acid coding for a IncW2 protein, an allele of which is depicted by SEQ ID N° 2.

2. The nucleic acid construct of claim 1, wherein said promoter active in the BETL is not an imprinted promoter.

3. The nucleic acid construct of claim 1, wherein said promoter active in the BETL is the BETL9 promoter, the sequence of which is SEQ ID N° 1.

4. The nucleic acid construct of claim 1, comprising SEQ **ID** No: 3.

5. A host cell containing at least the nucleic acid construct of any of claims 1 to 4.

6. The host cell of claim 5, wherein said expression cassette is stably integrated within the genome of said host cell.

7. A transgenic plant, or a part of a transgenic plant comprising at least one cell according to claim 5 or 6.

8. The plant or part of a plant of claim 7, which is a cereal.

9. The plant or part of a plant of claim 8, wherein said plant is chosen among maize, wheat, barley and rice.

10. A method for obtaining a transgenic plant, comprising the steps consisting of:

a) transforming at least a plant cell or plant tissue with a vector containing the nucleic acid construct of any of claims 1 to 4;
b) cultivating the cell(s) or plant tissue thus transformed so as to generate a transgenic plant containing at least a cell which contains, in its genome, at least the nucleic acid construct of any of claims 1 to 4.

11. A method for obtaining a plant containing a transgene, wherein said transgene comprises the nucleic acid construct of any of claims 1 to 4, comprising the steps of

a) performing the method of claim 10 in order to obtain a transgenic plant, wherein said transgene comprises the nucleic acid construct of any of claims 1 to 4
b) crossing said transgenic plant with a plant line which does not contain said transgene (the receiver plant line)
c) selecting, among the progeny, plants that contain said transgene and that have a good genome ratio with regard to said receiver plant line,
d) back-crossing said selected plants with said receiver plant line
e) repeating steps c) and d) if necessary until a line isogenic with said receiving line (and containing said transgene) is obtained,
f) optionally, performing self-fertilization in order to obtain a plant homozygotic for said transgene.

12. A method for growing a plant, comprising the step of sowing a plant seed, wherein said plant seed contains the nucleic acid construct of any of claims 1 to 4 and growing plants from this sowed seed.

13. A method for increasing plant yield under normal conditions, comprising the step of sowing plant seeds, wherein said plant seeds contain the nucleic acid construct of any of claims 1 to 4 and growing plants from these sowed seeds, and wherein the yield obtained from said grown plants is increased as compared to the yield obtained from plants grown from seeds which do not contain the nucleic acid construct of any of claims 1 to 4 or the yield obtained from said grown plants is maintained as compared to the yield obtained from plants containing the nucleic acid construct of any of claims 1 to 4 and grown in normal conditions.

14. A method for increasing or maintaining plant yield under stressed conditions, comprising the step of sowing plant seeds, wherein said plant seeds contain the nucleic acid construct of any of claims 1 to 4 and growing plants from these sowed seeds, wherein the growing phase is made under stress conditions, and wherein the yield obtained from said grown plants is increased as compared to the yield obtained from plants grown from seeds which do not contain the nucleic acid construct of any of claims 1 to 4 or the yield obtained from said grown plants is maintained as compared to the yield obtained from plants containing the nucleic acid construct of any of claims 1 to 4 and grown in normal conditions.

15. A method for selecting a plant that can be used in a breeding process for obtaining a plant with improved yield comprising the step of selecting, in a population of plant, the plants containing the nucleic acid construct of any of claims 1 to 4.

16. A method for identifying a plant with improved yield comprising the step of identifying, in a population of plant, the plants containing the nucleic acid construct of any of claims 1 to 4.

**17.** The method of any of claims 10 to 16, wherein said plant is a cereal.

**18.** The method of claim 17, wherein said plant is maize.

**19.** The method of claim 17, wherein said plant is wheat.

| Year | 2011 | | | 2011 | | | 2011 | | | 2011 | | | 2011 | | | 2011 | | |
|---|---|---|---|---|---|---|---|---|---|---|---|---|---|---|---|---|---|---|
| Variable | Plant Density | | | Moisture | | | Yield | | | Plant Density | | | Moisture | | | Yield | | |
| Type of location | Yield | | | Yield | | | Yield | | | Drought | | | Drought | | | Drought | | |
| Number of replicate | 5 | | | 5 | | | 5 | | | 5 | | | 5 | | | 5 | | |
| **Level** | **M** | **%** | **P** | **M** | **%** | **P** | **M** | **%** | **P** | **M** | **%** | **P** | **M** | **%** | **P** | **M** | **%** | **P** |
| [Control Equiv]Control Equiv | 78697 | | 0.7269 | 16.5 | | 0.7917 | 72.2 | | 0.7332 | 82238 | | 0.8674 | 15.8 | | 0.6989 | 65.0 | | 0.7358 |
| [Control Bulk-NS/Control Bulk-NS | | | | | | | | | | | | | | | | | | |
| [Control T00567/Control T00567 | 78672 | | | 16.3 | | | 74.3 | | | 82490 | | | 15.9 | | | 66.6 | | |
| [Control T01674/Control T01674 | 79647 | | | 16.7 | | | 72.8 | | | | | | | | | | | |
| [T 00246]T 00246_005 | 75093 | 95.0 | 0.0099 | 16.4 | 99.3 | 0.7227 | 72.3 | 98.9 | 0.8635 | 81022 | 98 | 0.3740 | 15.8 | 99 | 0.7217 | 69.9 | 106 | 0.3829 |
| [T 00246]T 00246_007 | 77331 | 97.9 | 0.2644 | 16.8 | 101.6 | 0.4605 | 72.5 | 99.1 | 0.8838 | 84172 | 102 | 0.2328 | 15.7 | 99 | 0.6253 | 72.5 | 110 | 0.1551 |
| [T 00246]T 00246_012 | 79042 | 100.0 | 0.9802 | 16.8 | 101.6 | 0.4605 | 68.7 | 94.0 | 0.3330 | 81878 | 99 | 0.7468 | 16.1 | 102 | 0.3393 | 69.7 | 106 | 0.4070 |
| [T 00246]T 00246_021 | 77820 | 98.5 | 0.4289 | 16.1 | 97.8 | 0.2967 | 75.1 | 102.7 | 0.6556 | 82535 | 100 | 0.9095 | 15.9 | 100 | 0.8741 | 72.5 | 110 | 0.1526 |
| [T 00246]T 00246_022 | 77699 | 98.3 | 0.5608 | 16.2 | 97.9 | 0.3240 | 80.2 | 109.7 | 0.1180 | 84149 | 102 | 0.2382 | 15.4 | 97 | 0.1327 | 74.9 | 114 | 0.0564 |
| [T 00246]T 00246_023 | 76188 | 96.4 | 0.0617 | 16.5 | 100.1 | 0.9617 | 71.4 | 97.7 | 0.7117 | 84113 | 102 | 0.2478 | 15.8 | 100 | 0.7877 | 67.5 | 103 | 0.7103 |
| [T 00246]T 00246_025 | 76994 | 97.5 | 0.1806 | 16.8 | 101.6 | 0.4605 | 79.5 | 108.7 | 0.1606 | 80403 | 98 | 0.1958 | 16.0 | 101 | 0.5170 | 69.6 | 106 | 0.4178 |

Figure 1

Europäisches Patentamt
European Patent Office
Office européen des brevets

# EUROPEAN SEARCH REPORT

Application Number
EP 14 30 5150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/093623 A1 (BIOGEMMA FR [FR]; PEREZ PASCUAL [FR]; PAUL WYATT [FR]; MUNIZ MENENDEZ) 23 August 2007 (2007-08-23)<br>* page 16, line 5 - page 17, line 13; example 3; sequence 18 *<br>----- | 1-19 | INV.<br>C07K14/415<br>C12N15/82 |
| X | WO 2007/057402 A1 (BIOGEMMA FR [FR]; PEREZ PASCUAL [FR]; CONSONNI GABRIELLA [IT]; PAUL WY) 24 May 2007 (2007-05-24)<br>* claim all; example 7.5; sequence 32 *<br>----- | 1-19 | |
| X | WO 2011/140329 A1 (CERES INC [US]; APUYA NESTOR [US]; SHARMA VIJAY [US]; WU CHUAN-YIN [US)<br>10 November 2011 (2011-11-10)<br>* claim all; sequence 2 *<br>----- | 1-19 | |
| X | Anonymous: "Overexpression of Maize Cell Wall Invertase Incw2 Gene in Maize Kernels",<br>Agricultural Science Paper,<br>25 February 2012 (2012-02-25),<br>XP055122587,<br>Retrieved from the Internet:<br>URL:http://www.agrpaper.com/overexpression-of-maize-cell-wall-invertase-incw2-gene-in-maize-kernels.htm<br>[retrieved on 2014-06-11]<br>* abstract *<br>----- | 1-19 | TECHNICAL FIELDS SEARCHED (IPC)<br>C07K<br>C12N |
| Y | WO 2010/129999 A1 (MOLECULAR PLANT BREEDING NOMIN [AU]; SPANGENBERG GERMAN [AU]; JOHN ULR)<br>18 November 2010 (2010-11-18)<br>* claim all; sequence 8 *<br>-----<br>-/-- | 1-19 | |

The present search report has been drawn up for all claims

| Place of search | Date of completion of the search | Examiner |
|---|---|---|
| Munich | 12 June 2014 | Marchesini, Patrizia |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 5150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | B.-H. KANG ET AL: "Miniature1-Encoded Cell Wall Invertase Is Essential for Assembly and Function of Wall-in-Growth in the Maize Endosperm Transfer Cell", PLANT PHYSIOLOGY, vol. 151, no. 3, 16 September 2009 (2009-09-16), pages 1366-1376, XP055122559, ISSN: 0032-0889, DOI: 10.1104/pp.109.142331 * abstract * ----- | 1-19 | |
| Y | TALIERCIO EARL W ET AL: "Isolation, characterization and expression analyses of two cell wall invertase genes in maize", JOURNAL OF PLANT PHYSIOLOGY, vol. 155, no. 2, August 1999 (1999-08), pages 197-204, XP002725715, ISSN: 0176-1617 * abstract * ----- | 1-19 | |
| | | | TECHNICAL FIELDS SEARCHED (IPC) |

The present search report has been drawn up for all claims

2

| Place of search | Date of completion of the search | Examiner |
|---|---|---|
| Munich | 12 June 2014 | Marchesini, Patrizia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 30 5150

This annex lists the patent family members relating to the patent documents cited in the above-mentioned European search report. The members are as contained in the European Patent Office EDP file on
The European Patent Office is in no way liable for these particulars which are merely given for the purpose of information.

12-06-2014

| Patent document cited in search report | Publication date | Patent family member(s) | Publication date |
|---|---|---|---|
| WO 2007093623 A1 | 23-08-2007 | AU 2007216465 A1 | 23-08-2007 |
| | | CA 2636890 A1 | 23-08-2007 |
| | | EP 1984389 A1 | 29-10-2008 |
| | | US 2009307795 A1 | 10-12-2009 |
| | | WO 2007093623 A1 | 23-08-2007 |
| WO 2007057402 A1 | 24-05-2007 | AU 2006314535 A1 | 24-05-2007 |
| | | CA 2629363 A1 | 24-05-2007 |
| | | EP 1948683 A1 | 30-07-2008 |
| | | US 2008313778 A1 | 18-12-2008 |
| | | WO 2007057402 A1 | 24-05-2007 |
| WO 2011140329 A1 | 10-11-2011 | US 2013125263 A1 | 16-05-2013 |
| | | WO 2011140329 A1 | 10-11-2011 |
| WO 2010129999 A1 | 18-11-2010 | AU 2010246909 A1 | 01-12-2011 |
| | | CA 2761533 A1 | 18-11-2010 |
| | | CN 102597237 A | 18-07-2012 |
| | | EP 2430160 A1 | 21-03-2012 |
| | | US 2012066795 A1 | 15-03-2012 |
| | | WO 2010129999 A1 | 18-11-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1999050427 A
- US 20090307795 A
- US 20080313778 A
- US 20120011621 A
- WO 0012733 A


### Non-patent literature cited in the description


- **SHEN et al.** *Maydica,* 2012, vol. 57, 147-153
- **WOO et al.** *The Plant Cell,* 2001, vol. 13, 2297-2317
- **HUGHES et al.** *Plant Biotechnol J. 2008 Oct,* 08 July 2008, vol. 6 (8), 758-69
- **ALTSCHUL et al.** *Nucleic Acids Res.,* 1997, vol. 25, 3389-3402
- **ALTSCHUL et al.** *FEBS J.,* 2005, vol. 272, 5101-5109
- **HUEROS.** *Plant Cell,* 1995, vol. 7, 747-757
- **MULLIS, K B.** *Methods in Enzymology,* 1987
- **ISHIDA et al.** *Nature Biotechnology,* 1996, vol. 14, 745-750